# FASCICULE DE BREVET EUROPEEN

(11) **EP 0 620 746 B1**
(45) Date de publication et mention de la délivrance du brevet: **10.04.1996**
(21) Numéro de dépôt: 93901612.7
(22) Date de dépôt: 13.01.1993
(51) Int. Cl.: A61M 1/00

(54) **DISPOSITIF D'ACCES RAPIDE A LA CIRCULATION SANGUINE**
VORRICHTUNG ZUM SCHNELLEN ANSCHLIESSEN AN DEN BLUTKREISLAUF
DEVICE FOR RAPIDLY ACCESSING CIRCULATING BLOOD

(30) Priorité: 09.11.1992 BR 9204347
(43) Date de publication de la demande: 26.10.1994
(73) Titulaire: GUFFI, Michele, Emilio, Alfredo, CH 6900 Lugano (CH); PEREIRA DE MAGALHAES, Helio, Sao Paulo, SP (BR)
(72) Inventeur: GUFFI, Michele, Emilio, Alfredo, CH 6900 Lugano (CH); PEREIRA DE MAGALHAES, Helio, Sao Paulo, SP (BR)
(74) Mandataire: Baggiolini, Raimondo Patent Attorneys
(86) Numéro de dépôt international: CH9300005
(87) Numéro de publication internationale: WO9411037

(56) Documents cités:
- EP-A- 0 078 565
- GB-A- 2 050 175
- US-A- 3 540 451
- US-A- 4 108 173
- US-A- 4 822 341

## Description

Cette invention se refère à un dispositif pour accès rapide à la circulation, afin de faciliter l'installation et la retraite des systèmes de support circulatoire des types substitutifs extracorporels (Voir par exemple Document US-A-3540451).

Le dispositif sert aussi bien à capter le sang, comme au retour du sang aux endroits où il est implanté.

Ses avantages résident dans l'application, la réapplication et la retraite de partie du système sans réouverture du thorax et sans anesthésie générale. Il existe aussi la diminution du risque d'infection, car les canules et les tubes utilisés peuvent être retirés sans ouverture du thorax, et les canules peuvent être réintroduites à tout moment, n'existant pas de solution de continuité entre la câge thoracique et l'extérieur.

Le coeur, organe central de l'appareil circulatoire, est un muscle creux, qui joue le rôle d'une pompe aspirante et comprimante remplissant ses 4 cavités (2 oreillettes et 2 ventricules) du sang qui provient des veines, en l'éjectant en parties égales dans l'artère pulmonaire et dans l'artère aorte, et, à travers de celles-ci, dans tout le réseau capillaire de l'organisme.

Du point de vue anatomique il est localisé dans l'espace intrathoracique, délimité latéralement par les poumons, antérieurement par l'os sternum, postérieurement par la colonne vertébrale et inférieurement par le diaphragme.

Du point de vue physiologique, les battements cardiaques vont définir le cycle cardiaque, lequel se subdivise en deux périodes (systole et diastole).

Schématiquement la diastole correspond à la période de remplissage des cavités et la systole à celle d'éjection sanguine en dehors des ventricules. Du point de vue anatomophysiologique le coeur se divise donc en une partie droite, responsable de l'aspiration du sang veineux pendant la diastole et de l'éjection de celui-ci dans la circulation pulmonaire grâce au ventricule droit, et en une partie gauche, qui reçoit le sang artérialisé provenant des poumons, qui sera ensuite poussé dans la circulation systémique par le ventricule gauche. La fonction principale des ventricules réside dans le maintien du débit cardiaque (=volume de sang éjécté par unité de temps) autour de 5 litres/minute.

Ce dernier ne dépend pas seulement de la contractilité du myocarde, mais aussi de la fréquence cardiaque, du degré de résistance périphérique à l'éjéction systolique (= post-charge) et du retour veineux (= pré-charge).

Les pressions au niveau de la circulation pulmonaire et systémique sont déterminées, respectivement, par le ventricule droit et gauche.

Les maladies du coeur peuvent être définies commes les pathologies qui affectent ses structures anatomiques, ses méchanismes biochimiques et physiologiques, en menant principalement à l'insuffisance cardiaque, c'est-à-dire à un état physiopathologique dans lequel une altération de la fonction cardiaque est responsable de l'incapacité du coeur de pomper le sang avec une fréquence proportionnelle aux besoins métaboliques des tissus périphériques et/ou de pouvoir faire cela seulement à partir d'une pression de remplissage élevée.

Lorsque le ventricule gauche n'arrive plus à entretenir un débit suffisant à maitenir la pression artérielle dans des valeurs normales, on est en présence du syndrome de bas débit cardiaque, qui est caractérisé par la diminution de l'irrigation de tous les organes, avec, par conséquent, détérioration des fonctions sous forme d'insuffisance rénale, vasculaire cérébrale, arrêt respiratoire, en aboutissant enfin à l'arrêt cardiaque avec décès si l'on n'instaure pas d'emblée les mesures thérapeutiques adéquates. Le ventricule droit peut aussi être compromis par l'insuffisance cardiaque, en provoquant des symptômes de bas débit pulmonaire. Si, malgré le traitement avec support pharmacologique maximal, malgré la correction des troubles métaboliques et le rétablissement correct de la volémie, l'index cardiaque (= débit cardiaque/surface corporelle) est inférieur à 1,8 l./min./m², la pression artérielle est inférieure à 90mm Hg, la diurèse est inférieure à 20ml/min. et s'il existe encore une tendance aux altérations métaboliques, on peut considérer que les critères hémodynamiques sont remplis pour introduire un système de support mécanique circulatoire.

Le support mécanique circulatoire ou circulation assistée constitue donc une mesure thérapeutique qui cherche d'un côté à diminuer le travail cardiaque et de l'autre côté à augmenter la nutrition cardiaque par les artères coronaires, aussi bien que celles de tous les organes, chez les patients en état de choc cardiogène après chirurgie cardiaque, chez les patients graves dans l'attente d'une transplantation cardiaque ou finalement chez les patients souffrant d'une détérioration aigue de la fonction cardiaque avec ou sans infarctus (= mort cellulaire locale du tissu myocardique, due dans la majorité des cas à l'occlusion d'une ou de plusieures artères coronaires).

Il existe deux grandes catégories de support mécanique qui se réfèrent d'un côté aux systèmes auxiliaires (ballon intra-aortique) et de l'autre aux systèmes substitutifs avec oxygénateur (dérivation artério-veineuse partielle) et sans oxygénateur (dérivation oreillette gauche-aorte ou oreillette droite-artère pulmonaire; ventricules artificiels externes et implantables, équipés de valves, pulsatils et alimentés par énergie électrique ou pneumatique).

La méthode de support mécanique la plus utilisée est constituée par la contrepulsation de sang artériel réalisée par un ballon mis en place dans l'artère aorte (=ballon intra-aortique = BIA). Celui-ci est insufflé et dégonflé d'après les battements cardiaques, c'est-à-dire qu'il est distendu pendant la diastole et dégonflé pendant la systole.

Les plus grands progrès liés au BIA sont à rechercher dans l'amélioration de la méthode de son introduction par voie périphérique transcutanée, en termes de facilité et de rapidité. Malheureusement ceci n'a pas contribué à diminuer l'incidence d'ischémie artérielle (= faute d'irrigation sanguine) des membres inférieurs, de dislocation de plaques d'atérosclérose et même de perforation de la paroi aortique.

Plusieurs patients qui développent un syndrome de bas débit cardiaque, son traités au début avec le BIA, mais, pour le fait de ne pas pouvoir augmenter le débit en dessus de 20%, il s'est révélé être une méthode insuffisante chez les patients graves, avec un index cardiaque inférieur à 1,5 l./min./m² et totalement inutile en cas d'asystolie (= absence de l'activité électrique du coeur).

La dérivation artério-veineuse partielle constitue un système de support circulatoire substitutif, qui permet le drainage et l'oxygénation du sang veineux à travers d'un oxygénateur et l'injection du sang artérialisé dans une artère périphérique. L'inconvénient principal de ce système est dû à l'anti-coagulation obligatoire, qui provoque régulièrement des complications hémorragiques graves. De plus, il faut mentionner qu'il est presque impossible de maintenir ce type d'assistance circulatoire au-delà de 48 heures. C'est en vertu de son application rapide et du rétablissement du débit cardiaque normal, que ce système permet d'évaluer l'état du patient et de tracer les directives de la thérapeutique à suivre.

Certains auteurs proposèrent l'aspiration du sang de l'oreillette gauche en utilisant une canule spéciale, introduite dans une veine du cou jusqu'à l'oreillette droite, passant ensuite à travers du septum interatrial (=structure anatomique qui sépare les deux oreillettes). Le sang aspiré était injecté dans une artère périphérique par une pompe synchronisée avec les battements cardiaques, afin d'éviter compétition de flux. Des problèmes liés à la technique et le risque de perforation atriale empêchèrent la continuation de ce système. Chez les patients qui nécessitent d'une assistance droite et gauche (= assistance biventriculaire), on peut employer des pompes centrifuges extracorporelles, qui sont reliées à la circulation sanguine par des canules de façon à recevoir le sang des oreillettes et à l'injecter dans l'artère pulmonaire et respectivement dans l'aorte.

Le désavantage de ces systèmes consiste dans la nécessité de laisser le thorax ouvert ou de pratiquer une nouvelle ouverture de celui-ci (= thoracotomie) pour extraire les canules et les conduits utilisés, après rétablissement de la fonction uni- ou biventriculaire.

Il a été proposé une autre technique, visant à suturer des canules spéciales à la paroi de l'aorte et de l'oreillette gauche pour permettre l'assistance ventriculaire gauche. Ces canules sont ensuite obturées par un guide occlusif et restent définitivement dans le thorax du patient. Les désavantages de ce système sont déterminés d'un côté par l'impossibilité de réintroduire la circulation assistée sans risque élevé de thromboembolisme et de contaminations sanguines directes, provoquées par la manipulation des obturateurs et de l'autre coté par la persistance de corps étrangers qui prédisposent à l'infection à l'endroit où ils se localisent.

Les dispositifs d'assistance mécanique temporaire ventriculaires pulsatils, alimentés par une source énergétique électrique ou pneumatique extracorporelle, peuvent se situer en dehors du thorax (= support uni- ou biventriculaire de Pierce-Donachy), dans l'abdomen (= dispositif Novacor , Thermocardiosystem etc.), ou diectement dans la câge thoracique (= coeur artificiel total type Jarvik 7 ).

Tous ces dispositifs possèdent des valves internes et peuvent substituer le coeur droit, gauche ou les deux.

Pour faire face aux problèmes sus-mentionnés, il a été développé un dispositif, objet de cette invention, qui permet un accès rapide à la circulation sanguine, facilitant l'installation et la retraite des systèmes de support circulatoire des types substitutifs extracorporels.

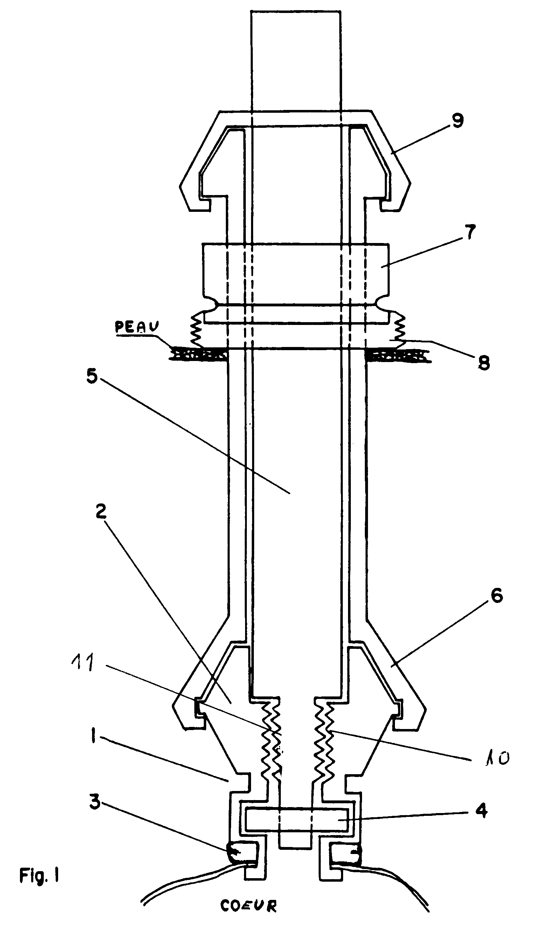

La figure ci-jointe (FIG.1) permettra une meilleure compréhension de l'invention et représente une vue frontale en coupe longitudinale.

Le dispositif de cette invention est constitué d'une pièce implantable (1) dans la paroi atriale gauche et droite, dans le tronc de l'artère pulmonaire ou dans l'artère aorte, composée d'une structure rigide et tubulaire (2) équipée d'un rebord en polyester texturisé (3) pour suture dans les structures anatomiques. Cette pièce contient un diaphragme (4) d'élastomère avec fentes, pour permettre le passage d'une canule (5) pour drainer ou injecter le sang.

La canule en plastique (5) avec pointe en plastique est munie d'un filetage et entourée par un tube amovible flexible (6) d'élastomère, qui connecte la pièce implantée (1) avec la peau du patient.

Le tube amovible (6) est équipé d'un tube en plastique rigide (7), muni à sa fois d'un rebord en polyester (8); ce tube (6) est equipé enfin d'un bouchon en plastique (9) de protection et d'isolement.

En anesthésie générale on pratique l'ouverture du thorax et on aborde directement la structure anatomique où il faut appliquer le dispositif, soit au niveau du coeur, soit au niveau des grands vaisseaux de la base (= artère pulmonaire et artère aorte). Après avoir fait une suture circulaire et une incision dans le tissu, on introduit la pièce implantable (1), qui est fixée par la suture circulaire, serrée par un tourniquet. Cette fixation permet de suturer de manière hémostatique le rebord de polyester (3) avec le tissu. Au travers d'un orifice thoracique (= thoracostomie) on effectue le passage du tube amovible (6) jusqu'à obtenir son emboîtement avec la partie externe de la structure tubulaire rigide (2), implantée précédemment dans le tissu.

Le tube en plastique rigide (7) peut glisser en dehors du tube amovible (6) et il est équipé d'un rebord de polyester (8), lequel est suturé à la peau.

Dans le tube amovible (6) est insérée la canule (5) du dehors en dedans du thorax, jusqu'à entrer en contact avec l'écrou de la pièce implantée (1). En vissant la canule (5) dans cette dernière, on obtient l'ouverture du diaphragme (4) avec par conséquent accès direct au système circulatoire.

Dès que le dispositif est implanté, il est connecté avec une pompe extracorporelle, qui permet l'aspiration du sang à partir de l'endroit où est situé le dispositif. Le sang sera ensuite injecté au niveau d'une artère périphérique ou au niveau des grands vaisseaux de la base, dans lesquels serait implanté le même dispositif selon la technique décrite ci-dessus, lequel constituerait l'extrémité efférente intrathoracique de la circulation assistée. Ce système ne nécessite d'aucune anticoagulation, mais seulement d'une prophylaxie antithrombotique avec des petites doses d'héparine (= médicament qui empêche la formation de caillot sanguin).

L'arrêt de la circulation assistée est reproduit en dévissant les canules (afférente, efférente ou les deux) de façon à permettre la fermeture du diaphragme interne.

Pour entrer de nouveau en circulation assistée, il suffit de visser très rapidement la (les) canule(s) du dehors du thorax. Pour retirer le(s) tube(s) amovible(s), il suffit de le(s) livrer de la peau en coupant la suture cutanée du polyester. Avec une main on immobilise la canule vissée dans la pièce implantée dans le tissu et on exerce une traction du dehors sur le tube amovible jusqu'à provoquer sa dislocation. Le tube amovible étant disloqué, on dévisse la (les) canule(s) comme décrit précédemment et on retire les deux ensemble par simple traction.

## Revendications

1. DISPOSITIF D'ACCES RAPIDE A LA CIRCULATION SANGUINE comprenant une pièce implantable (1) constituée d'une structure rigide tubulaire (2), munie d'un rebord en polyester texturisé (3), pour suture dans les structures anatomiques et contenant, à son intérieur, un diaphragme (4) d'élastomère, avec fentes, et un écrou pour permettre le passage d'une canule en plastique (5) avec pointe en plastique munie d'une vis, ladite structure (2) étant entourée par un tube amovible flexible (6) d'élastomère, qui connecte ladite pièce (1) avec la peau du patient; ledit tube (6), étant équipé d'un segment cylindrique en plastique rigide (7) avec un rebord en polyester (8), et étant équipé aussi d'un bouchon en plastique (9) de protection et d'isolement.

## Claims

1. DEVICE FOR RAPID ACCESS TO THE BLOOD CIRCULATORY SYSTEM, comprising an implantable member (1) constituted by a rigid tubular structure (2), provided with a texturized polyester border (3), for sewing into the anatomical structures and containing, within it, an elastomeric diaphragm (4), with slots and a nut, to permit the passage of a plastic canula (5) with a plastic point provided with a thread, the said structure (2) being surrounded by a removable flexible tube (6) of elastomer, which connects the said member (1) with the skin of the patient; said tube (6) being provided with a cylindrical segment of rigid plastic (7) with a polyester border (8) and being provided also swith a plastic plug (9) for protection and insulation.

## Patentansprüche

1. VORRICHTUNG FÜR EINEN SCHNELLEN ZUGANG ZUM BLUTKREISLAUF, mit einem implantierbaren Teil (1), das aus einer rohrförmigen, starren Struktur (2) gebildet ist, die mit einer texturierten Polyestereinfassung (3) versehen ist, um in die anatomischen Strukturen genäht zu werden, und in seinem Inneren eine elastomere Membran (4) mit Schlitzen sowie eine Mutter enthält, um die Durchführung einer mit einer Schraube versehenen Kunststoff-Kanüle (5) mit Kunststoff-Spitze zu ermöglichen, wobei die Struktur (2) von einem flexiblen, lösbaren elastomeren Rohr (6) umgeben ist, das das Teil (1) mit der Haut des Patienten verbindet; wobei das Rohr (6) mit einem zylindrischen Segment aus starrem Kunststoff (7) und mit einer Polyestereinfassung (8) ausgerüstet ist und außerdem mit einem Schutz- und Isolierstopfen (9) aus Kunststoff versehen ist.
